# EUROPEAN PATENT SPECIFICATION

(11) **EP 2 163 247 B1**
(45) Date of publication and mention of the grant of the patent: **20.08.2014**
(21) Application number: 08772950.5
(22) Date of filing: 11.06.2008
(51) Int. Cl.: A61K 31/352, A61K 31/7048, A61P 43/00, A61P 35/00, A61P 35/04, A61P 1/16, A61P 11/00

(54) **USE OF NARINGENIN AND NARINGIN AS INHIBITORS FOR TRANSFORMING GROWTH FACTOR-beta1 SIGNALING PATHWAY**
VERWENDUNG VON NARINGENIN UND NARINGIN ALS INHIBITOREN ZUR UMWANDLUNG DES WACHSTUMSFAKTOR-BETA1-SIGNALSTOFF-STOFFWECHSELGANG
UTILISATION DE NARINGÉNINE ET DE NARINGINE EN TANT QU'INHIBITEURS POUR LA TRANSFORMATION D'UNE VOIE DE SIGNALISATION DU FACTEUR DE CROISSANCE béta1

(30) Priority: 13.06.2007 CN 200710118871
(43) Date of publication of application: 17.03.2010
(73) Proprietor: The Institute of Biophysics Chinese Academy of Sciences, Beijing 100101 (CN); Henan Topfond Pharmaceutical Co., Ltd., Henan 463000 (CN)
(72) Inventor: LIANG, Wei, Beijing 100101 (CN); DU, Gangjun, Beijing 100101 (CN); ZHANG, Hongyan, Beijing 100101 (CN); JIN, Lingtao, Beijing 100101 (CN); LV, Heping, Henan 463000 (CN); WANG, Wei, Henan 463000 (CN); LIU, Jinping, Henan 463000 (CN); JIAO, Guohua, Henan 463000 (CN)
(74) Representative: Chapman, Paul Gilmour
(86) International application number: PCT/CN2008/001129
(87) International publication number: WO 2008/151519

(56) References cited:
- WO-A2-01/51043
- WO-A2-2004/042020
- WO-A2-2006/128169
- XINGJUN LIU ET AL: "Smad3 Specific Inhibitor, Naringenin, Decreases the Expression of Extracellular Matrix Induced by TGF-[beta]1 in Cultured Rat Hepatic Stellate Cells" PHARMACEUTICAL RESEARCH, KLUWER ACADEMIC PUBLISHERS-PLENUM PUBLISHERS, NE LNKD- DOI:10.1007/S11095-005-9043-5, vol. 23, no. 1, 1 January 2006 (2006-01-01), pages 82-89, XP019370960 ISSN: 1573-904X
- CAMPBELL, J.K. ET AL.: "Synergistic effects of flavonoids on cell proliferation in Hepa-1c1c7 and LNCaP cancer cell lines" JOURNAL OF FOOD SCIENCE, vol. 71, no. 4, 2006, pages S358-S363, XP002584031
- LEE, M. ET AL.: "The flavonoid naringenin inhibits dimethylnitrosamine-induced liver damage in rats" BIOL. PHARM. BULL., vol. 27, no. 1, 2004, pages 72-76, XP009133999
- SYU-ICHI KANNO ET AL: "Inhibitory effect of narigenin on tumour growth in human cancer cell lines and sarcoma S-180-implanted mice" BIOLOGICAL & PHARMACEUTICAL BULLETIN (OF JAPAN), PHARMACEUTICAL SOCIETY OF JAPAN, TOKYO, JP, vol. 28, no. 3, 3 March 2005 (2005-03-03), pages 527-530, XP009133998 ISSN: 0918-6158 [retrieved on 2004-12-28]
- DATABASE CA [Online] 2006 LIU X. ET AL.: 'Smad3 Specific Inhibitor, Naringenin, Decreases the Expression of Extracellular matrix Induced by TGF-beta1 in Cultured Rat Hepatic Stellate Cells', XP008117272 Database accession no. (144:266856) & PHARMACEUTICAL RESEARCH vol. 23, no. 1, 2006, pages 82 - 89
- ZHAO W. ET AL.: 'The effect of versulin and naringenin on cell proliferation of hepatic stellate cells and anabolic effect of collagen thereof' CHINESE HEPATOLOGY (CHINESE) vol. 10, no. 3, September 2005, pages 226 - 227
- DATABASE CA [Online] 2005 KANNO S.-I. ET AL.: 'Inhibitory effects of naringenin on tumor growth in human cancer cell lines and sarcoma S-180-implanted mice', XP008117274 Database accession no. (143:613) & BIOLOGICAL & PHARMACEUTICAL BULLETIN vol. 28, no. 3, 2005, pages 527 - 530
- DATABASE CA [Online] 2005 TOTTA P. ET AL.: 'Mechanisms of Naringenin-induced Apoptotic Cascade in Cancer Cells: Involvement of Estrogen Receptor alpha and beta Signalling', XP008117273 Database accession no. (142:86072) & IUBMB LIFE vol. 56, no. 8, 2004, pages 491 - 499
- DATABASE CA [Online] 2006 CAMPBELL J.K. ET AL.: 'Synergistic effects of flavonoids on cell proliferation in Hepa-1c1c7 and LNCaP cancer cell lines', XP008117213 Database accession no. (145:202218) & JOURNAL OF FOOD SCIENCE vol. 71, no. 4, 2006, pages S358 - S363
- JIA D. ET AL.: 'Advance in Research of Physiologically-active Compounds in Pummelo Peel' FOOD AND FERMENTATION INDUSTRIES (CHINESE) vol. 27, no. 11, 2001, pages 74 - 78, XP008124961

## Description

### Technical Field

The invention relates to the use ofnaringenin and naringin, in particular their use as inhibitors for transforming growth factor-β1 (TGF-β1) signaling pathway for use in the treatment and/or prevention of lung fibrosis and lung tumor metastasis.

### Background Art

Fibrosis is the common pathological basis for a variety of chronic diseases, and a major intermediate step for sclerosis of tissues and organs and even for tumorigenesis. Fibrosis mainly refers to a pathological process in which various pathogenic factors stimulate cytoclasis in tissues and viscera, and extracellular matrix (ECM) abnormally increases and overly deposits in tissues. It is characterized by proliferation and precipitation of a large amount of fibrous tissues, and 4~7-fold increased collagen content [Won-I1 Jeong et al, Hepatology, 44( 6):1441-1451, (2006)]. During fibrosis of tissues and organs, fibroblasts are the bases for various presentation of local fibrosis. Activated fibroblasts have a very strong ability to produce and recombine collagen. Some cytokines in tissue damage and inflammation occurrence, such as cellular adhesion molecule 1, vascular endothelial growth factor, connective tissue growth factor etc. modulate the activation of fibroblasts [Jurgen J. W. et al, Annals of Surgery, 242(6):880-887,(2005)]. Many studies showed that TGF-β played a critical role in a fibrosis process, especially in a pathological fibrosis process. TGF-β not only directly activates the transformation from static fibroblasts into myofibroblasts, but also induces production of PDGF and expression of the receptors thereof on fibroblasts, indirectly stimulates proliferation and activation of fibroblasts, promotes synthesis of extracellular matrix such as collagen, fibronectin and proteoglycan etc. from fibroblasts, and increases ECM synthesis [Victorino R. Briones et al, Biochem Biophys Res Commun, 345(2):595-601, (2006)]. In addition, TGF-β results in a failure of normal degradation of ECM by downregulating MMP expression, and also suppresses the degradation and accelerates the accumulation of ECM by upregulating the expression of PAI and TIMP, thereby ultimately leading fibrosis to take place. Therefore, inhibition of TGF-β may prevent and treat fibrosis diseases [Christelle Guyot et al, The International Journal of Biochemistry & Cell Biology 38: 135-151, (2006)].

TGF-β is not only a key regulator for fibrosis formation, but also a very strong modulator for growth, differentiation and migration of cells. It also plays an important role in occurrence, development and metastasis of tumors. Clinic studies have demonstrated that the over-expression of TGF-β1 was at least associated with pathogenesis of tumors such as human breast cancer, rectal cancer, esophageal cancer, stomach cancer, liver cancer, lung cancer, pancreatic cancer, particularly the over-expression of TGF-β1 was related to the development and metastasis of tumors, neovascularization and the poor prognosis [Brian Bierie et al, Cytokine & Growth Factor Reviews, 17:29-40, (2006)]. TGF-β plays a certain extent inhibitory action to tumor growth at the beginning oftumorigenesis, but subsequently turns to promoting tumor development, which is shown in 3 aspects: 1) making cells to lose control on growth inhibition; 2) enabling tumor cells to achieve metastasis; 3) enabling tumor cells to escape immunosurveillance. The major mechanism of enabling tumor cells to escape immunosurveilance is that TGF-β1 inhibits the production of interferon-y (IFN-γ) in different ways during the beginning and memory phases of CD4⁺ T cells. Therefore, suppression of TGF-β may also prevent and treat tumor diseases [Brian Bierie et al, Nature Reviews/Cancer, 6 :506-520, (2006)].

For the past a few years, emergence of the concept of cancer related fibroblasts led us to notice the correlation between fibrotic conditions and tumorigenesis [Akira Orimo et al, Cell Cycle, 5(15): 1597-1601, (2006)]. By investigation, the present inventors found that fibrosis provided a suitable microenvironment for tumor development and TGF-β signaling pathway played an important role in that process.

Currently, TGF-β signaling pathway-targeted therapies include altered immuno-components, small molecule inhibitors, soluble proteins and anti-sense complex inhibitors. However, there is still no TGF-β signaling pathway -interfering medicament in the market, and there is no medicament ideal for treating fibrosis clinically, except for classic recombinant IFN-γ [Eiji Suzuki et al, Cancer Res, 67(5): 2351-2359(2007)].

Flavanoids are effective ingredients commonly existing in Chinese traditional medicines. They have a variety of pharmacological functions, such as anti-bacteria, anti-inflammation, free radical cleaning, and anti-tumor, etc [Mouming Zhao et al, International Immunopharmacology, 7: 162-166,(2007)].

Naringin, also named aurantiin, has a formula of C₂₇H₃₂O₁₄, molecular weight of 580.53, mainly exists in pericarp and pulp of Rutaceae, such as grapefruit, tangerine and oranges, and is also the major effective ingredient in Chinese herbal medicine such as fortune's drynaria rhizome, *Fructus Aurantii Immaturus, Fructus Aurantii seu ponciri, Exocarpium Citri Rubrum.* It has been reported that subcutaneous injection of 100mg/kg naringin results in an evident anti-inflammation function, and 200mg/L naringin strongly suppresses vesicular stomatitis viruses in rats. Naringin can also lower the viscosity of blood, reduce the formation of thrombi, have analgesic and sedative abilities, as well as a stronger function for increasing the secretion of bile for experimental animals. Naringin also have many features such as desensitizating, anti-sensitivity, promoting blood circulation, relieving muscular spasm, improving topical micro-circulation and supplying nutrition, thus resulting in some unique curative effects, such as promoting the evacuation of medicines, eliminating the streptomycin-caused injure for the No. 8 pair of brain nerve, and relieving noxious side effects of streptomycin [Bok, S. H et al, Nutr. Res. 20, 1007-1015(2000)].

Naringenin, aglycone of naringin, is the pharmacologically functioning structural moiety of naringin. Naringenin has a formula of C₁₅H₁₂O₅, molecular weight of 272.25, and also has functions such as anti-bacteria, anti-inflammation, anti-spasm, and cholagogic effect, etc [Chul-Ho Lee et al, Biochemical and Biophysical Research Communications 284, 681-688 (2001)]. The structures ofnaringenin and naringin are as follows:

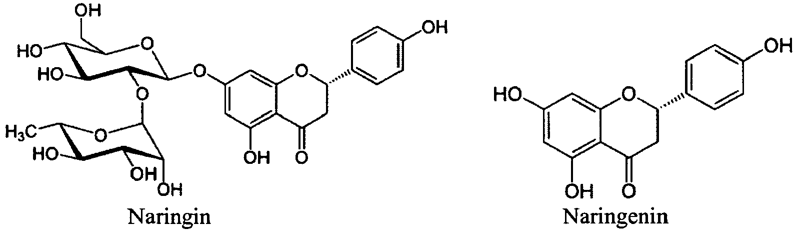

WO 01/51043 discloses the use of naringenin, alone or in combination with other natural agents in the treatment and prevention of cancer, such as lung cancer and liver cancer by way of killing cancer cells.

### Disclosure of the Invention

The present inventors firstly found that natural flavonoids small molecule compounds, i.e. naringenin and naringin, commonly existing in lemons, grape juice and oranges, etc, could inhibit TGF-β signaling pathway, thereby raising serum level of IFN-γ. Both in vivo and in vitro experiments demonstrated that naringenin and naringin possess therapeutic and prophylactic functions to fibrosis and tumors. The potential clinic application of naringenin and naringin was further investigated intensely.

The detailed disclosure of the invention is as follows:
(1) The use of naringenin and naringin as inhibitors of transforming growth factor-β1 signaling pathway, for preparing medicament to treat or prevent lung fibrosis or lung tumor metastasis.
(2) The use according to (1), wherein the effective dose range of said inhibitors of transforming growth factor-β1 signaling pathway to treat or prevent fibrosis for the animal is 10-300mg/kg body weight/day orally, 4-200mg/ kg body weight/day by subcutaneous injection, 1-30mg /kg body weight/day by intravenous injection, preferably 50-200mg/kg body weight/day orally, 10-100mg/kg body weight/day by subcutaneous injection, 5-20mg/kg body weight/day by intravenous injection; their putatively effective dose range for human is 0.8-24mg/kg body weight/day orally, 0.2-6mg/kg body weight/day by subcutaneous injection, 0.8-4mg/kg body weight/day by intravenous injection, preferably 4-12mg/kg body weight/day orally; 0.6-2mg/kg body weight/day by subcutaneous injection; 0.1-0.4mg/kg body weight/day by intravenous injection.

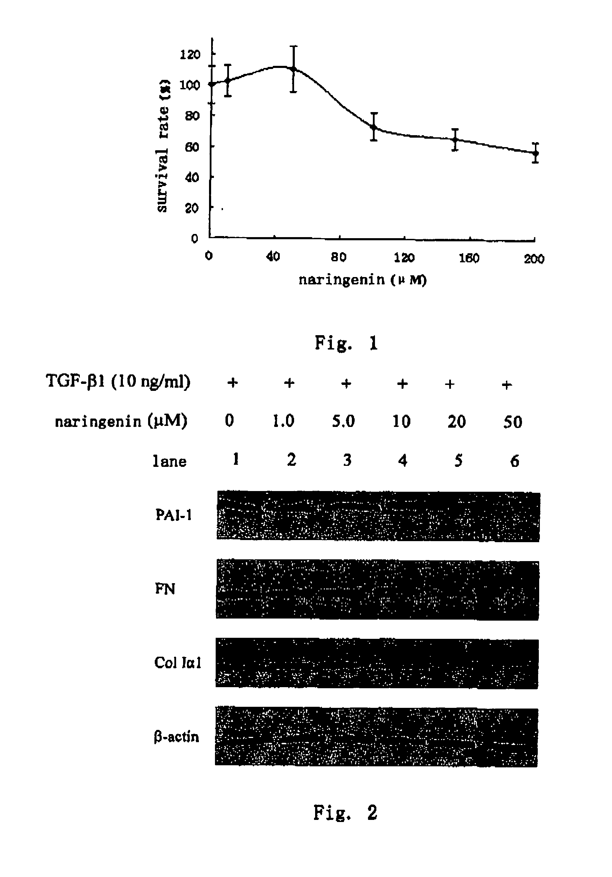

The invention discovers that naringenin and naringin can be used for treating or preventing lung fibrosis and lung tumor metastasis as inhibitors of TGF-β1 signaling pathway. By way of background naringenin inhibits the proliferation of hepatic stellate cells of the rats and the secretion of cellular matrix and collagen thereof (see figure 1 and figure 2). Naringenin dramatically inhibits the proliferation of hepatic stellate cells, and significantly suppresses the expression of plasminogen activation inhibitor 1 (PAI-1), fibronectin(FN) and collagen 1 α (Co 1 Ialpha1) in rats. The results described above indicate that naringenin possesses a potential anti-fibrosis function.

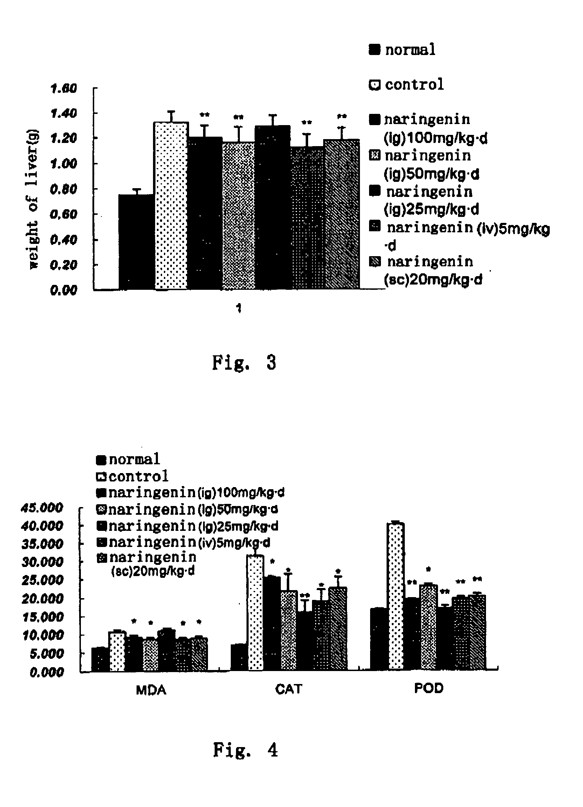

Naringenin performs a protective function to the hepatic damage (fibrosis) caused by carbon tetrachloride (CCl₄). The size of a liver directly reflects the damaging tumefaction extent of the liver. High and medium doses of naringenin clearly inhibit the extension of livers in the mice with CCl₄-caused hepatic fibrosis (p<0.01, see figure 3). MDA (malondialdehyde) is the product after peroxidatically damaging lipid in organisms. The higher content of MDA indicates the larger damaging extent of the organisms. High, medium and low doses of naringenin evidently reduce the serum level of MDA in the mice with CCl₄-caused hepatic fibrosis (p<0.05, see figure 4) ; CAT (catalase) and POD (peroxidase) eliminate H₂O₂, and reduce self damaging in organisms. The increase of H₂O₂ concentration in vivo is the essential factor for raising the activities of CAT and POD. High, medium and low doses of naringenin all reduce the serum excitabilities of CAT and POD in the mice with CCl₄-caused hepatic fibrosis (p<0.05, see figure 4). The data shows naringenin reduces free radicals in vivo.

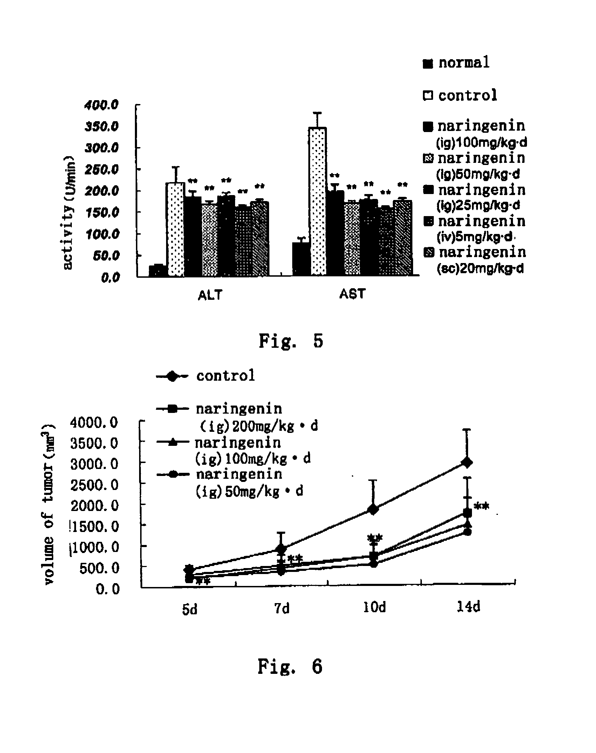

The activities of ALT (Alanine aminotransferase) and AST (Aspartate aminotransferase) reflect the damaging extent of livers directly, wherein the higher activities indicate the larger damage to livers. High, medium and low doses of naringenin all reduce the increase of serum ALT and AST levels in the mice with CCl₄-caused hepatic fibrosis (p<0.01, see figure 5. The above results indicate that naringenin performs an evident protective function to the hepatic damage (fibrosis) caused by CCl₄ in mice.

In addition, therapies with 3 dosages of naringenin, i.e. 200, 100, 50mg/kg body weight/day orally, 20, 10, 5mg/kg body weight/day by intravenous injection, or 40, 20, 10mg/kg body weight/day by subcutaneous injection, and the same mole dosage of naringin all significantly inhibit the growth of mouse hepatic cancer H22 subcutaneous tumors (P<0.01, see figures 6, 7 and 8).

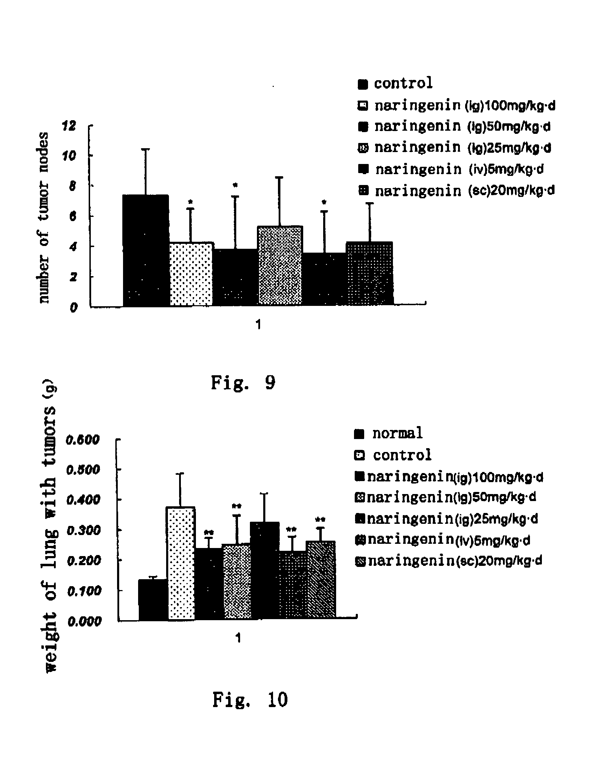

The three dosage groups of naringenin all can lessen the pulmonary metastasis of Lewis lung cancer (P<0.05, see figure 9) and slow down the growth of pulmonary metastatic tumors (P<0.01, see figure 10), indicating that naringenin possesses a function of anti-metastasis of tumors.

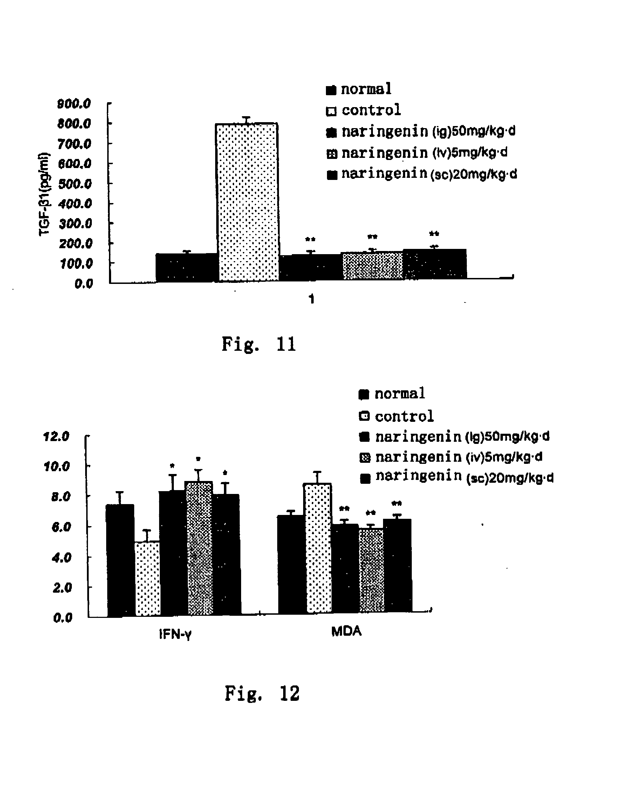

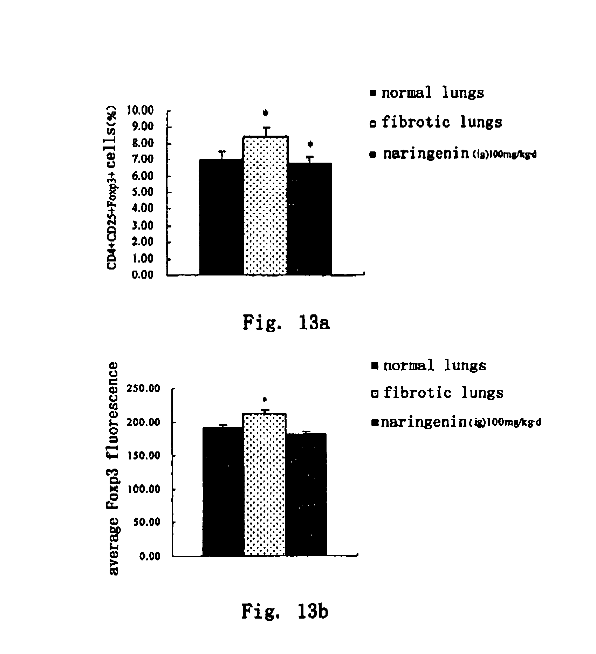

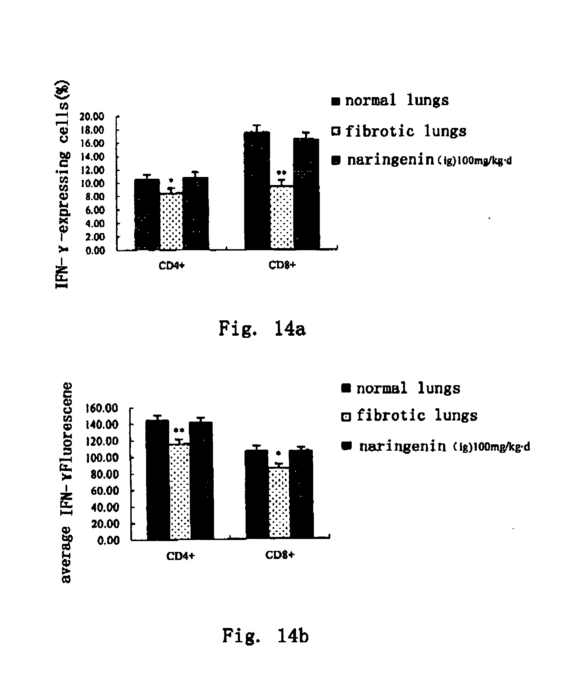

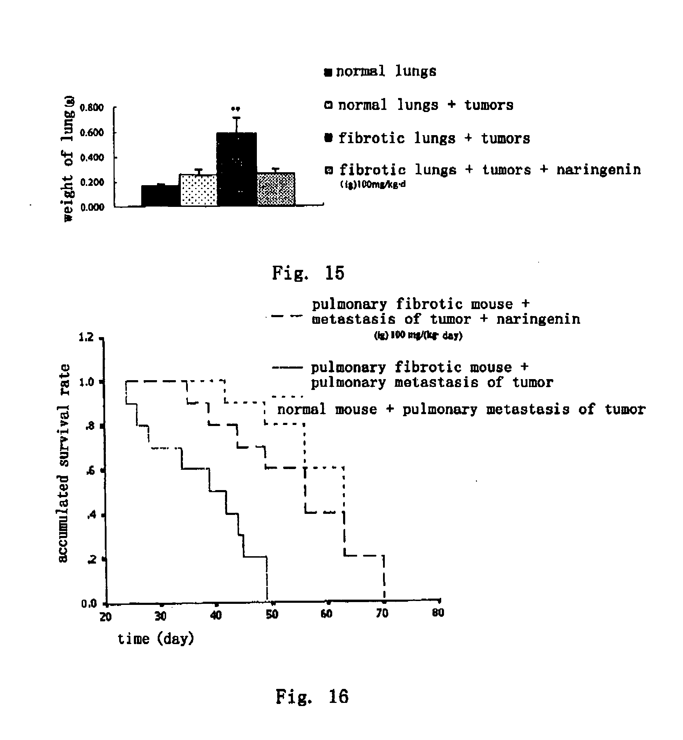

Naringenin significantly suppresses the serum level of TGF-β1 (P<0.01, see figure 11), and remarkably raises the serum level of IFN-γ (P<0.05, see figure 12) in the mice with bleomycin-induced pulmonary fibrosis. Naringenin prevents the increase of lung tissue Treg in the mice with bleomycin-induced pulmonary fibrosis (P<0.05, see figure 13), and thus raises IFN-γ back up in the lung tissue of the mice with pulmonary fibrosis (P<0.05, see figure 14). Naringenin also inhibits pulmonary metastasis of tumors in the mice with bleomycin-induced pulmonary fibrosis (P<0.01, see figure 15), so as to prolong the survival period of the mice with pulmonary fibrosis after pulmonary tumors have metastasized (P<0.01, see figure 16). Those results indicate that naringenin can treat fibrosis and tumor diseases by reducing TGF-β1 level and inducing production of IFN-γ both in serum and in tisssues. Also, naringenin reduces the serum level of MDA in mice (P<0.01, see figure 12). Taken together, in view of the above disclosure, the beneficial effects of the invention can be summarized in the following aspects:
1) Naringenin and naringin perform therapeutic or prophylactic functions to the bleomycin-caused pulmonary fibrosis in mice, thereby evidently lessening the extent of pulmonary fibrosis, and preventing lungs from being damaged.
2) Naringenin and naringin clearly inhibit metastasis of Lewis lung cancer in mice, thereby reducing the incidence of metastasis of Lewis lung cancer, and restraining the growing rate of pulmonary metastatic tumors.
3) Naringenin and naringin significantly lower the increased levels of serum TGF-β1 and MDA caused by fibrosis in mice, thereby regulating them back to normal, and also, naringenin clearly brings the decreased level of serum IFN-γ causey by TGF-β1 increasing back up.

### Brief Description of the Drawings

Figure 1 is a curve graph showing the influence of naringenin on proliferation of hepatic stellate cells in rats.
Figure 2 is a graph showing the influence of naringenin on expression and secretion of cellular matrix collagenous protein including plasminogen activation inhibitor1(PAI-1), fibronectin(FN) and collagen1α (Co1 Ialpha1).
Figure 3 is a histogram showing the influence of naringenin on tumefaction of livers in the mice with CCl₄-caused hepatic fibrosis, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 4 is a histogram showing the influence of naringenin on the increased levels of serum MDA, CAT and POD in the mice with CCl₄-caused hepatic fibrosis, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 5 is a histogram showing the influence of naringenin on the increase of serum ALT and AST levels in the mice with CCl₄-caused hepatic fibrosis, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 6 is a curve chart showing the influence of naringenin (ig) on growth of mouse hepatic cancer H22 subcutaneous tumors, wherein Nar(ig) represents intragastric administration of naringenin or naringin.
Figure 7 is a curve chart showing the influence of naringenin (iv) on growth of mouse hepatic cancer H22 subcutaneous tumors, wherein Nar(iv) represents intravenous injection of naringenin or naringin.

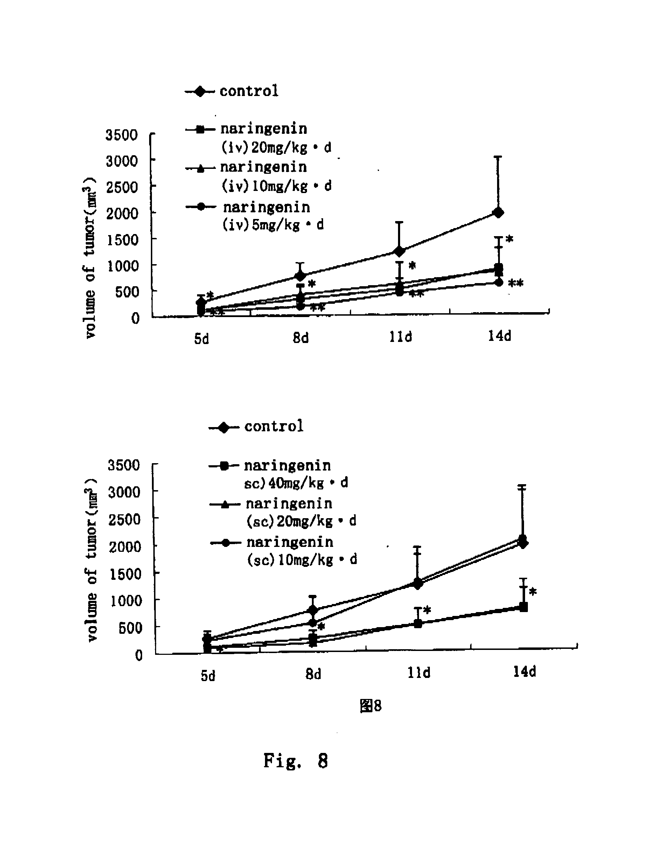

Figure 8 is a curve chart showing the influence of naringenin (sc) on growth of mouse hepatic cancer H22 subcutaneous tumors, wherein Nar(sc) represents subcutaneous injection of naringenin or naringin.
Figure 9 is a histogram showing the influence ofnaringenin on metastasis of Lewis lung cancer in mice, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 10 is a histogram showing the influence of naringenin on growth of tumors after metastasis of Lewis lung cancer in mice, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 11 is a histogram showing the influence of naringenin on the increase of serum TGF-β1 level in the mice with bleomycin-induced pulmonary fibrosis, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 12 is a histogram showing the influence of naringenin on the bleomycin-induced decrease of serum IFN-γ level and increase of serum MDA level in mice, wherein Nar(ig) represents intragastric administration of naringenin or naringin, Nar(iv) represents intravenous injection (iv) of naringenin or naringin, and Nar(sc) represents subcutaneous injection (sc) of naringenin or naringin.
Figure 13 is a histogram showing the influence of naringenin on the increase of lung tissue Treg in the mice with bleomycin-induced pulmonary fibrosis, wherein figure 13a presents Treg cell percentage, figure 13b presents an average intensity (fluorescence) of cells expressing Foxp3. Nar(ig) represents intragastric administration of naringenin.
Figure 14 is a histogram showing the influence of naringenin on the decrease of lung tissue IFN-γ in the mice with bleomycin-induced pulmonary fibrosis, wherein figure 14a is a cell percentage histogram showing the IFN-γ expression of CD4+ and CD8+ lymphocyte of lung tissues in mice, figure 14b is an average intensity (fluorescence) histogram showing the IFN-γ expression of CD4+and CD8+ lymphocyte of lung tissues in mice, Nar(ig) represents intragastric administration of naringenin.
Figure 15 is a histogram showing the influence of naringenin on pulmonary metastasis of tumors in the mice with bleomycin-induced pulmonary fibrosis, wherein Nar(ig) represents intragastric administration of naringenin.
Figure 16 is a curve graph showing the influence ofnaringenin on survival period of the mice with pulmonary fibrosis and pulmonary metastatic tumors, wherein Nar(ig) represents intragastric administration of naringenin.

### Mode of Carrying Out the Invention

Naringenin and naringin of the invention can be used as inhibitors in TGF-β1 signaling pathway, so as to treat or prevent lung fibrosis or lung tumor metastasis. Other disclosures are provided by way of background. The anti-fibrosis and anti-tumor activities of naringenin and naringin were tested in the invention. Naringin has functions comparable to those of naringenin with the same mole dose, so only naringenin was demonstrated in the following examples. Naringenin and naringin of the invention were both commercially purchased from Shanxi Huike Botanic Development Ltd.

### Example 1. The influence of naringenin on the proliferation of hepatic stellate cells and the secretion of extracellular matrix in rats.

**Methods:** Rat hepatic stellate cells (HSC-T6, Hepatic stellate cell, provided by Prof. Friedman, The Medical Center of Mount Sinai, USA) were cultured in the RPMI1640 medium (Sigma Co.) containing 10% fetal calf serum (PPA Co., USA), 100 U/ml of each of penicillin and streptomycin, at 5%CO₂, 37°C (FIL-TER model incubator, Thermo Co. Germany) . After the cells with 90% abundance were digested with 0.25% trypsin, the number of cells was adjusted to 5 × 10⁴/ml. The cells were inoculated into a 96-well culture plate, with 100 µl/well. The cells were adherently cultured overnight, and then the medium containing naringenin with different concentrations was added into the plate, with 100 µl/well. There were 4-6 replicate wells for each concentration. After the cells were cultured another 48h, the medium was removed. Phosphate buffered saline (PBS) containing 0.5mg/ml MTT (dimethylthiazol-2-yl)- 2,5- diphenyltetrazolium bromide (Sigma Co.) was added into the wells, with 100 µl/well. After 4h culturing, MTT was removed, and dimethyl sulfoxide (DMSO), 100 pl/well, was added. The absorbance was measured at 590 nm by an enzyme photometer (model MK3, Thermo Co., Germany). The absorbance of the control well (without naringenin) was 100%. The ratio between absorbance of the treated well and the control well is the percentage of survived cells. The influence of naringenin on the proliferation of rat hepatic stellate cell was observed from the plotted cell survival curve. To investigate the influence of naringenin on the secretion of eatraxellular matrix in rat hepatic stellate cells, 2 × 10⁵ cells /ml were inoculated into a 6-well plate, 3 replicate wells for each well. The treating procedure was same as the above 96-well plate. After 48h treatment, cells were digested with trypsin, washed with PBS for 3 times. Subsequently, collagen1α (Col Ialpha1, collagen Iα1), fibronectin(FN) and plasminogen activation inhibitor1(PAI-1, plasminogen activator inhibitor-1) were detected by Western blotting method. The human recombinant cytokine TGF-β1 used in this example was purchased from R&D Co., USA.

The protein maker and protein ladder were purchased from Bio-Rad Co., USA. All antibodies (including primary and secondary antibodies) were purchased from Santa Cruz Biotech. Co., USA.

**Results:** The activated proliferation of hepatic stellate cells was the basis for forming hepatic fibrosis, and naringenin evidently inhibited the proliferation of hepatic stellate cells in rats (p<0.05, see figure 1). Plasminogen activation inhibitorl(PAI-1), fibronectin(FN) and collagen1α (Col lalpha1), secreted by activated stellate cells, were the matrix materials required for fibrosis. These three types of extracelular matrices were down-regulated by naringenin (see figure 2). The above results indicated that naringenin possessed an anti-fibrosis function.

### Example 2. The influence of naringenin on hepatic fibrosis caused by carbon tetrachloride (CCl₄) in mice

**Methods:** Female balb/c mice (provided by Beijing Weitonglihua Experimental Animal Co.) were s.c. injected with 10% CCl₄ (carbon tetrachloride), 0.2ml/animal, twice per week, for consecutive 4 weeks, so as to establish the hepatic fibrosis model. From week 2, the treated groups were intragastrically administrated (ig) with high (100mg/kg), medium (50mg/kg) and low (25mg/kg) dosages of naringenin (Nar), or intravenously injected (iv) with 5mg/kg naringenin (Nar) and subcutaneously injected (sc) with 20mg/kg naringenin (Nar). Another group of mice without establishing the fibrosis model was used as normal control mice. There were 10 mice in each group, and they were dosed once daily, for consecutive 3 weeks. On day 28, the blood samples were taken from orbital veins and detected for blood index. Then, the mice were sacrificed, and the livers were taken out and weighted. In this example, MDA, CAT and POD kits were purchased from Nanjing Jiancheng Biotech. Co., China; ALT and AST kits were purchased from Beijing Zhongshengbeikong Biotech. Co., China.

**Results:** The size of livers directly reflected the damaging tumefaction extent of the liver. High and medium dosages ofnaringenin clearly inhibited extension of the livers in the mice with CCl₄-caused hepatic fibrosis; and iv and sc administrated naringenin were effective as well (p<0.01, see figure 3). MDA (malondialdehyde) was the product after peroxidatically damaging lipid in organisms. The higher content of MDA indicated the larger damaging extent of the organisms. High, medium and low dosages of naringenin evidently reduced the serum level of MDA in the mice with CCl₄-caused hepatic fibrosis; and iv and sc administrated naringenin were effective as well (p<0.05, see figure 4) . CAT (catalase) and POD (peroxidase) eliminated H₂O₂, and reduced self damaging in organisms. The increase of H₂O₂, concentration in vivo was the essential factor for raising the activities of CAT and POD. High, medium and low dosages of naringenin all reduced the serum excitabilities of CAT and POD in the mice with CCl₄-caused hepatic fibrosis; and iv and sc administrated naringenin were effective as well (p<0.05, see figure 4), which indirectly demonstrated naringenin reduced free radicals in vivo. The activities of ALT (Alanine aminotransferase) and AST (Aspartate aminotransferase) reflected the damaging extent of livers directly, wherein the higher activities indicated larger damages to livers. High, medium and low dosages of naringenin all reduced the increase of serum ALT and AST levels in the mice with CCl₄-caused hepatic fibrosis; and iv and sc administrated naringenin were effective as well (p<0.01, see figure 5) . The above results indicated that naringenin performed an evident suppressing function to the hepatic fibrosis caused by CCl₄.

### Example 3. The influence of naringenin on the growth of mouse hepatic cancer H22 subcutaneous tumors

**Methods:** The mouse hepatic cancer H22 subcutaneous tumors (provided by Institute of Materia Medica, Chinese Academy of Medical Sciences) were suspended into PBS to make cell suspension. After the number of cells was adjusted to 5 × 10⁶/ml, the cell suspension was subcutaneously inoculated into the axillae of anterior limb of female balb/c mice, with 0.2 ml/animal. From the next day following tumor inoculation, the treated groups were intragastrically administrated (ig) with 200mg/kg, 100mg/kg and 50mg/kg doses of naringenin (Nar), intravenously injected (iv) with 20mg/kg, 10mg/kg and 5mg/kg doses of naringenin (Nar) or subcutaneously injected (sc) with 40mg/kg, 20mg/kg and 10mg/kg doses of naringenin (Nar). There were 10 mice in each group, and they were dosed once daily, for consecutive 2 weeks. After tumorigenesis, the size of tumors were measured using a vernier caliper every 3 days, and the volume of tumors was calculated as length × width × 0.5 width, so as to plot a tumor growth curve.

**Results:** ig or iv administrated high, medium and low dosages of naringenin and sc administrated high and medium dosages of naringenin inhibited the growth of mouse hepatic cancer H22 subcutaneous tumors (P<0.01 or P<0.05, see figures 6. 7 and 8), indicating that naringenin has an anti-tumor activity.

### Example 4. The influence of naringenin on metastasis of Lewis lung cancer

**Methods:** The mouse Lewis pulmonary cancer subcutaneous tumors (provided by Institute of Materia Medica, Chinese Academy of Medical Sciences) were suspended into PBS to make cell suspension. After the number of cells was adjusted to 1 × 10⁶/ml, the cell suspension was iv inoculated into female balb/c mice, with 0.2ml/animal. From the next day following tumor inoculation, the treated groups were intragastrically administrated (ig) with high (100mg/kg), medium (50mg/kg) and low (25mg/kg) dosages ofnaringenin (Nar), intravenously injected (iv) with 5mg/kg naringenin (Nar) or subcutaneously injected (sc) with 20mg/kg naringenin (Nar) . There were 10 mice in each group, and they were dosed once daily, for consecutive 3 weeks. On day 22 following tumor inoculation, the mice were sacrificed, and the lungs were taken out and weighted. The number of visible tumor nodes was counted.

**Results:** High and medium dosages ofig administrated naringenin lessened the metastasis of Lewis lung cancer (P<0.05, see figure 9) and slow down the growth of pulmonary metastatic tumors (P<0.01, see figure 10), and iv or sc administrated naringenin were effective as well, indicating that naringenin possesses a function of anti-metastasis of tumors.

### Example 5. The influence of naringenin on serum levels of TGF-β1, IFN-γ and MDA in the mice with bleomycin-caused pulmonary fibrosis

**Methods:** Female balb/c mice were anaesthetized with 30mg/kg pentobarbital sodium (0.3%, 0.2ml/animal), and the pulmonary fibrosis model was established by administrating 100µg bleomycin/animal (products of the Cosmetics and Medication Corporation, Japan, 2µg/ml, 50µl/animal) by one-time intranasal dripping. From the next day following tumor inoculation, the treated groups were treated with naringenin (Nar) ig (50mg/kg), iv (5mg/kg), or sc (20mg/kg). There were 10 mice in each group, and they were dosed once daily, for consecutive 3 weeks. On day 22 following the establishment of model, blood was sampled and sera were extracted, so as to detect TGF-β1, IFN-γ and MDA using a ELISA kit. TGF-β1 and IFN-γ kits used in this example was provided by Genzyme Co., USA.

**Results:** TGF-β1 (transforming growth factor β1) was the major cytokine for the formation of fibrosis, and also the promotor of tumorigenesis and development of tumor. IFN-γ (interferon-γ) had an contrary function to TGF-β1, thus reducing fibrosis and preventing tumor growth. All of the 3 administration pathways ofnaringenin clearly inhibited the increase of serum TGF-β1 level (P<0.01, see figure 11), and promoted IFN-γ secretion (P<0.05, see figure 12) in the mice with bleomycin-induced pulmonary fibrosis, which indicated naringenin was able to treat fibrosis and tumor diseases by inhibiting TGF-β1 β1 and inducing IFN-γ. Also, the decreased serum MDA level resulted by naringenin in mice (P<0.01, see figure 12) was another piece of evidence showing naringenin could prevent damaging for organisms.

### Example 6. The influence of naringenin on the lung tissue Treg and IFN-γ in the mice with bleomycin-induced pulmonary fibrosis

**Methods:** The bleomycin-induced mouse pulmonary fibrosis models were used to construct pulmonary fibrotic female Balb/c mice. Also, 10 normal control mice were constructed by being parellelly treated with intranasal dripping of physiological saline. In other words, the distribution of mice was as follows: 1) normal control (Normal): intranasally dripped with physiological saline, intragastrically administered with 0.5% CMC-Na; 2) model control (Control): intranasally dripped with bleomycin, intragastrically administered with 0.5% CMC-Na; 3) naringenin-treated group (Narl00mg/kg): intranasally dripped with bleomycin, intragastrically administered with 100mg naringenin suspended in 0.5% CMC-Na /kg body weight. There were 20 mice in each group. On the first day of the experiment, a mouse pulmonary fibrosis model was established by intranasally dripping of bleomycin. On Day 2, the treated group was given with naringenin by intragastric administration; the untreated group was given with same volume of solvent (0.1ml/10g body weight) by intragastric administration, once daily for both. On Day 21, the mice were sacrificed, and the lungs were excised. Following being cut, the tissue was digested in HBSS solution containing 1 mg/ml collagenase D and 0.02mg/ml DNase I at 37°C for 90 min, to become single cells. The sample was filtered with a 70µm cell filter to remove cell debris, washed with HBSS solution twice, resuspended into 35% Percoll PBS, loaded into the top part of 70% Percoll, and centrifuged at 2500 rpm for 20 min. The lymphocytes were collected, cultured with the stimulation of 25ng/ml PMA and 500ng/ml Ionomycin for 5h, and then cultured another 3h after Brefeldin A solution was added. After fixed and permeated according to the instruction of the kit, the cells were stained with FITC-coupled anti-CD4, Percp-cy5.5- coupled anti-CD8, and PE-coupled anti-IFN-γ. Then, the ratio and strength of IFN-γ expression were selected for subgroups of quantitative lymphocytes by a flow cytometer. The ratio and strength of intracellular Foxp3 expression in the subgroup ofCD4+CD25+ lymphocytes were performed according to the instruction of the Regulatory T Cell Staining kit.

**Results:** Inhibitory T regulatory cell (Treg) was the major regulator of immuno-suppression, and also the executor of TGF-β1 immuno-suppression. Twenty-one days after modeling with bleomycin, the proportion of CD4+/CD25+ cells expressing Foxp3, an Treg marker, was increased in the fibrotic pulmonary tissues of mice (P<0.05, see figure 13a, 13b), and the corresponding IFN-γ level were both decreased in CD4+ and CD8+ (P<0.05, see figure 14a, 14b). Ig treating with 100mg naringenin /kg brought the changes in the fibrotic pulmonary tissues of mice back to normal (P<0.05, see figure 13, 14), which indicated naringenin could abolish the immuno-suppression in the fibrotic pulmonary tissues by inhibiting TGF-β1.

### Example 7. The influence of naringenin on the mice with pulmonary fibrosis and pulmonary metastatic tumors

**Methods:** The bleomycin-induced mouse pulmonary fibrosis models were used to construct 40 pulmonary fibrotic female Balb/c mice. Also, 20 normal control mice were constructed by being parellelly treated with intranasal dripping of physiological saline. In other words, the distribution of mice was as follows: 1) normal control (Normal): intranasally dripped with physiological saline, intragastrically administered with 0.5% CMC-Na; 2) model control (Control): intranasally dripped with bleomycin, intragastrically administered with 0.5% CMC-Na; 3) naringenin-treated group (Narl00mg/kg): intranasally dripped with bleomycin, intragastrically administered with 100mg naringenin suspended in 0.5% CMC-Na/kg body weight. Seven days after intranasal dripping of bleomycin, the mouse breast cancer 4T1 cells were cultured in vitro and digested with trypsin, in order to generate single cell suspension. After the number of cells was adjusted to 5×10⁵/ml, 0.2ml of the cell suspension/animal was inoculated into each mouse by injecting through tail veins. On the same day, the treated group was given with naringenin by intragastric administration; the untreated group was given with same volume of solvent (0.1ml/10g body weight) by intragastric administration, once daily, for consecutive 4 weeks for both. On Day 28 following tumor inoculation, 10 mice/group were sacrificed, and the lungs were taken out and weighted. The number of visible tumor nodes of tumor was counted, and the incidence of pulmonary metastasis of tumors and the size of metastatic tumors were compared. Another 10 mice were tested for survival periods. The number of mice died of pulmonary metastasis within 70 days following tumor inoculation was recorded, and analyzed for survival of the mice.

**Results:** Seven days after modeling with bleomycin, the mice were i.v. inoculated with the tumor cells. Twenty-eight days after the mice with pulmonary fibrosis was inoculated with tumors, the weight of lungs with tumors was 0.584g, which was 2.3-fold heavier than that of the control mice without pulmonary fibrosis (0.254g). After the mice with pulmonary fibrosis was ig treated with 100mg naringenin/kg, the weight of lungs with tumors was 0.263g, which was decreased by 54.95% compared to that of the untreated mice with pulmonary fibrosis, and comparable to that of the control mice without pulmonary fibrosis (P>0.05, see figure 15).

During the observation of survival periods, the median survival was 39 days for the 10 pulmonary metastatic mice with pulmonary fibrosis, which was 24 days shorter than the median survival (63 days) of the 10 pulmonary metastatic mice without pulmonary fibrosis (38.1%). After the 10 pulmonary metastatic mice with pulmonary fibrosis was ig treated with 100mg naringenin/kg, the median survival was 56 days, which was 17 days longer than the median survival of the untreated pulmonary metastatic mice with pulmonary fibrosis (43.6%) (P<0.01, see figure 16).

## Claims

1. The use of naringenin and naringin as inhibitors of transforming growth factor-β1 signaling pathway for the preparation of a medicament for the treatment and/or prevention of lung fibrosis.

2. The use of naringenin and naringin as inhibitors of transforming growth factor-β1 signaling pathway for the preparation of a medicament for the treatment and/or prevention of lung tumor metastasis.

3. The use according to claim 1 or 2, wherein the medicament is to be administered orally, by subcutaneous injection, or by intravenous injection.

4. Naringenin and naringin as inhibitors of transforming growth factor-β1 signaling pathway for use in the treatment and/or prevention of lung fibrosis.

5. Naringenin and naringin as inhibitors of transforming growth factor-β1 signaling pathway for use in the treatment and/or prevention of lung metastatic tumors.

## Patentansprüche

1. Verwendung von Naringenin und Naringin als Inhibitoren des Transformations-Wachstumsfaktor-β1-Signalwegs zur Herstellung eines Medikaments für die Behandlung und/oder Verhütung von Lungenfibrose.

2. Verwendung von Naringenin und Naringin als Inhibitoren des Transformations-Wachstumsfaktor-β1-Signalwegs zur Herstellung eines Medikaments für die Behandlung und/oder Verhütung von Lungentumor-Metastase.

3. Verwendung nach Anspruch 1 oder 2, wobei das Medikament oral, durch subkutane Injektion oder durch intravenöse Injektion verabreicht wird.

4. Naringenin und Naringin als Inhibitoren des Transformations-Wachstumsfaktor-β1-Signalwegs zur Verwendung bei der Behandlung und/oder Verhütung von Lungenfibrose.

5. Naringenin und Naringin als Inhibitoren des Transformations-Wachstumsfaktor-β1-Signalwegs zur Verwendung bei der Behandlung und/oder Verhütung von Lungentumor-Metastase.

## Revendications

1. Utilisation de naringénine et de naringine en tant qu'inhibiteurs de la voie de signalisation du facteur de croissance transformant β1, pour la préparation d'un médicament pour le traitement et/ou la prévention d'une fibrose pulmonaire.

2. Utilisation de naringénine et de naringine en tant qu'inhibiteurs de la voie de signalisation du facteur de croissance transformant β1 pour la préparation d'un médicament pour le traitement et/ou la prévention de la métastase d'une tumeur du poumon.

3. Utilisation selon la revendication 1 ou 2, dans laquelle le médicament doit être administré oralement, par injection sous-cutanée ou par injection intraveineuse.

4. Naringénine et naringine en tant qu'inhibiteurs de la voie de signalisation du facteur de croissance transformant β1, pour l'utilisation dans le traitement et/ou la prévention d'une fibrose pulmonaire.

5. Naringénine et naringine en tant qu'inhibiteurs de la voie de signalisation du facteur de croissance transformant β1, pour l'utilisation dans le traitement et/ou la prévention de tumeurs métastatiques du poumon.
